## Europäisches Patentamt

## European Patent Office

## Office européen des brevets

(11) Veröffentlichungsnummer: **0 174 448**
**B1**

(12) **EUROPÄISCHE PATENTSCHRIFT**

(45) Veröffentlichungstag der Patentschrift:
**01.03.89**

(51) Int. Cl.⁴: **G 01 N 31/22**, G 01 N 33/00

(21) Anmeldenummer: **85107570.5**

(22) Anmeldetag: **19.06.85**

(54) Verfahren zum Nachweis von allergenhaltigem Hausstaub.

(30) Priorität: **22.08.84 DE 3430896**

(43) Veröffentlichungstag der Anmeldung:
**19.03.86 Patentblatt 86/12**

(45) Bekanntmachung des Hinweises auf die Patenterteilung:
**01.03.89 Patentblatt 89/9**

(84) Benannte Vertragsstaaten:
**AT BE CH FR GB IT LI LU NL SE**

(56) Entgegenhaltungen:
FR-A- 2 502 786

CHEMICAL ABSTRACTS, Band 102, Nr. 15, 15. April 1985, Seite 296, Nr. 128184e, Columbus, Ohio, US; E. BISCHOFF et al.: "Color test for allergenic house dust. Part 2. Supplementary aspects" & ALLERGOLOGIE 1985, 8(1), 36-8

(73) Patentinhaber: **Werner & Mertz GmbH, Ingelheimstrasse 1-3, D-6500 Mainz 1 (DE)**

(72) Erfinder: **Bischoff, Edelbert, Dr., Leibnizstrasse 52, D-6719 Kirchheim-Bolanden (DE)**
Erfinder: **Schirmacher, Wolfgang, Dipl.-Chem., Rilkeallee 28, D-6500 Mainz 31 (DE)**

(74) Vertreter: **Zumstein, Fritz, Dr. et al, Dr. F. Zumstein sen Dr. E. Assman Dipl.-Ing. F. Klingseisen Dr. F. Zumstein jun. Bräuhausstrasse 4, D-8000 München 2 (DE)**

## Beschreibung

Dem Auftreten von Hausstaubmilben (Dermatophagoides pteronyssinus, farinae usw,) wird in neuerer Zeit zunehmend Aufmerksamkeit gewindet (G. Hoffmann in «Luftqualität in Innenräumen» Gustav Fischer Verlag Stuttgart - New York 1982, Seite 385 bis 401). Grund dafür ist, daß festgestellt wurde, daß ein beträchtlicher Teil der Bevölkerung — man rechnet mit etwa 5% — unter asthmatischen Erscheinungen leidet, die auf Bestandteile des Hausstaubes zurückzuführen sind.

Bei diesen Bestandteilen handelt es sich hauptsächlich um den Kot der o.g. Hausstaubmilben sowie um Bruchstücke abgestorbener Milben. Im Darmtrakt der lebenden und abgestorbenen Milben sind die die Gesundheitsstörungen auslösenden Allergene enthalten (E. Petri et al in Allergologie, Jg. 5, Nr. 3/1982 Seite 109 bis 119). Eine lebende Milbe selbst enthält nur etwa ein Tausendstel der Allergenmenge, die sie in der Zeit ihres Lebens erzeugt. Der Kot selbst ist vielfach von Pilzen besiedelt, die ihrerseits ebenfalls Allergene hervorbringen können. Die Hauptmenge der Allergene befindet sich in den Staubablagerungen, und zwar auch dann noch, wenn die ursprüngliche Milbenpopulation durch Bekämpfungsmassnahme abgetötet bzw. reduziert wurde.

Aufenthaltsorte der Hausstaubmilben sind Polstermöbel, Betten, Fußböden und Wände, insbesondere wenn sie mit textilen Materialien überzogen sind. Flächenmässig gesehen dürften Teppichböden die grösste Rolle spielen.

Von den Fußböden und Polstermöbeln wird durch Begehen und Benutzen Feinststaub aufgewirbelt, der sich oft an unzugänglichen Stellen, die weitgehend frei von Luftzug sind, z.B. hinter Schränken, ansammelt. Auch beim Staubsaugen selbst wird dieser Feinststaub z.T. aufgewirbelt und gelangt so auch an Stellen, die selbst nicht von Milben besiedelt sind. In allen Fällen führt erneutes Aufwirbeln zu erneuten Belästigungen der Bewohner der betreffenden Räume.

Die Beseitigung der Ursachen für die Hausstaub-Allergie hat zur Voraussetzung, daß die Ursache erkannt wird. Denn es gibt auch andere Allergien, z.B. gegen Pollen (Heuschnupfen). Die Hausstaub-Allergie belästigt jedoch die davon betroffenen Menschen das ganze Jahr über, da Milbenkot und Milbenreste praktisch ununterbrochen vorhanden sind. Arzt und Patienten stehen deshalb immer wieder vor der Frage, ob ein Milbenbefall vorliegt.

Mit der europäischen Patentanmeldung 84 113 705.2 EP-A-0 144 820, veröffentlicht am 19.06.85 sowie der weiteren europäischen Patentanmeldung 85 101 299.7, EP-A-0 152 068 veröffentlicht am 21.08.95, wurde der Weg zur Lösung der Aufgabe beschrieben, ein einfaches Verfahren zum Nachweis allergenhaltiger Milben-Rückstände zu finden. Aufgrund der vorhandenen aromatischen Diazo-Verbindung, z.B. Diazosulfanilsäure, bildet sich bei Anwesenheit von Milben-Exkrementen eine ziegelrote Färbung, die, wie die Anmelderin festgestellt hat, in ihrer Intensität proportional zu der in der Staubprobe vorhandenen Milbenkot-Konzentration ist.

In Weiterführung dieser Arbeiten hat sich nun ergeben, daß der in der EP-A-0 144 820 beschriebene Weg zur Beurteilung der Milbenkot-Konzentration auch geeignet ist für die Beurteilung der allergologischen Gesamtaktivität einer Staubprobe. Damit ist es nicht nur möglich, allergenhaltigen Hausstaub durch eine Farbreaktion qualitativ und halbquantitativ nachzuweisen, sondern darüber hinaus auch eine Aussage über die Belastung durch Allergene zu machen, die in diesem Hausstaub enthalten sind.

Bestätigt wird dies durch einen Vergleich der Meßergebnisse nach dem erfindungsgemässen Verfahren mit derjenigen Testmethode, die, dem Stand der Technik entsprechend, derzeit als die wirksamste anzusehen ist, d.h. mit dem Radio-Allergo-Sorbens-Test (RAST).

Die statistische Auswerung der Untersuchungsergebnisse, die an gleichen Staubproben sowohl mit RAST als auch dem hier beschriebenen Verfahren (mit Hilfe der Farbreaktion) gewonnen wurden, sowie deren Bezug auf die mikroskopisch kontrollierte Milbenpopulation erbringt einen hohen Grad von Übereinstimmung in der Beurteilung der allergologischen Gesamtaktivität, wobei die Auswerung nach der Spearman-Correlation-Coefficient-Methode erfolgte [Sidney Siegel, «Nonparametric Statistics», McGraw Hill Intern. Book Comp., Auckland (1956)].

Im Vergleich mit RAST ist das erfindungsgemässe Verfahren einfacher durchzuführen, da die betreffende Staubprobe lediglich extrahiert werden muß und die Farbintensität dann direkt im Extrakt gemessen werden kann. Die Methode nach RAST ist bereits vom Konzept her aufwendiger und unterliegt in ihren einzelnen Schritten der ihr gemässen Problematik; so wird versucht, von der Indizierung mit radioaktiven Substanzen wieder wegzukommen (s. dazu D. Nolte «Allergiediagnostik», Dustri-Verlag, Karl Feistle, München-Deisenhofen (1981), insbesondere Seite 29 ff. sowie M. Werner & V. Ruppert «Praktische Allergiediagnostik», Georg Thieme-Verlag, Stuttgart (1979), Seite 96 ff.).

Diese neuen Befunden der Anmelderin über die Beurteilung der Farbreaktion veranlaßten die vorliegende Weiterbildung der EP-A-0 144 820, nämlich eine quantitative Messung.

Einige bevorzugte Ausführungsformen des erfindungsgemässen Verfahrens werden nachstehend wiedergegeben:

Bevorzugt wird die zu untersuchende Staubprobe vorher durch Behandeln mit einem erwärmten wäßrig-alkoholischen Lösemittelgemisch von nachweisstörenden Bestandteilen befreit.

Aus der verbleibenden Staubprobe werden die nachweisrelevanten Bestandteile durch Extraktion mit einer wäßrig-alkoholischen Alkalimetallhydroxid-Lösung abgetrennt. Einzelheiten finden sich in der europäischen Patentanmeldung 84 113 705.2.

Mit Hilfe von Nachweispapieren, z.B. gemäss europäischer Patentanmeldung 85 101 299.7 läßt sich an einer Probe des erhaltenen Extrakts als Vorversuch feststellen, welche Farbintensität zu erwarten ist. Einzelheiten finden sich in dieser europäischen Patentanmeldung 85 101 299.7.

Je nach Intensität wird ein Anteil zwischen 1 und 10 ml des Extrakts entnommen, mit Sodalösung und anschliessend mit einer geeigneten aromatischen Diazo-Verbindung, z.B. Diazosulfanilsäure, versetzt. Einzelheiten bezüglich der aromatischen Diazoverbindungen finden sich in der EP-A-0 144 820.

Nach etwa 20 Minuten Farbentwicklung wird wäßrig-alkoholisches Lösemittelgemisch zugefügt und die so erhaltene Farblösung spektralphotometrisch gegen eine Blindprobe gemessen.

Das Meßergebnis wird mit Hilfe einer Guanin-Eichkurve ausgewertet.

Die Ausgestaltung des hier beschriebenen Verfahrens im einzelnen kann von dem jeweils damit befaßten fachkundigen Analytiker durchgeführt werden. Nur beispielhaft, jedoch nicht obligatorisch, sei folgender Weg genannt:

*Beispiel*

1. Einwaage
   Einwagage: 250 mg allergenhaltigen Hausstaubes.

2. Reinigung
   Die Staubprobe wird im Zentrifugenglas von 10 ml Inhalt mit 5 ml «Lösung I» digeriert, die zuvor auf 60°C erwärmt wurde. Nach Zentrifugieren und Abdekantieren wird der Vorgang wiederholt.

3. Extraktion
   Die noch feuchte vorgereinigte Staubprobe wird im Zentrifugenglas mit 3 ml «Lösung II» durchmischt und dann zentrifugiert. Mit 3 ml «Lösung I» wird nachgewaschen.

4. Farbentwicklung
   Die so erhaltene Nachweislösung einschliesslich Waschlösung wird filtriert und in einem 100 ml Meßkolben mit dest. Wasser aufgefüllt. Von dieser Lösung werden 1 bis 10 ml (je nach zu erwartender Farbintensität entsprechend Vorversuch) in einen 25 ml Meßkolben pipettiert, 3 ml 20%ige Sodalösung und 2 ml 0,05 %ige wässrige Diazosulfanilsäurelösung zugefügt. Nach 20 Minuten werden 10 ml «Lösung I» zugegeben und dann mit dest. Wasser aufgefüllt.

5. Farbmessung
   Die Messung erfolgt bei 490 Nanometer gegen eine Blindwertprobe. Die Auswertung geschieht anhand einer Eichkurve mit Guanin. (Hierzu stellt man verschiedene Lösungen von Guanin (in «Lösung II») mit einem Gehalt zwischen 20 µg und 100 µg in mehreren 25 ml Meßkolben her. Die Farbentwicklung erfolgt entsprechend der bei Ziff. 4 angegebenen Vorschrift für die Staubprobe.

Lösung I:  20 Volumenteile Methanol und 100 Volumenteile Wasser;
Lösung II: 1,9 g KOH in 200 ml «Lösung I».

**Patentansprüche**

1. Verfahren zur Feststellung des Vorkommens von allergenhaltigem Hausstaub, wie er sich bei oder nach Auftreten von Hausstaubmilben ergibt, bei dem man Proben von in Gebäuden vorkommendem Staub in wäßrig-alkoholischer Alkalimetallhydroxid-Lösung gründlich suspendiert, mit dem entstehenden Extrakt eine Farbreaktion mit Hilfe einer aromatischen Diazo-Verbindung durchführt, dadurch gekennzeichnet, dass man zur Beurteilung der allergologischen Aktivität der Staubprobe eine quantitative Messung der Farbintensität der Farbreaktion vornimmt.

2. Verfahren nach Anspruch 1, dadurch gekennzeichnet, dass die Farbmessung mit Hilfe eines Spektralphotometers durchgeführt wird.

3. Verfahren nach Anspruch 1 und 2, dadurch gekennzeichnet, dass die eingesetzte Staubprobe, zum Zweck der Reinigung, vorher mit einem wäßrig-alkoholischen Gemisch vorbehandelt worden ist.

4. Verfahren nach Anspruch 1 bis 3, dadurch gekennzeichnet, daß man die für die Extraktion der nachweisrelevanten Bestandteile stark alkalische Lösung im Interesse der nachfolgenden Farbentwicklung auf die Minimalmenge beschränkt.

5. Verfahren nach Anspruch 1 bis 4, dadurch gekennzeichnet, daß man den nach Anspruch 4 erhaltenen stark alkalischen Extrakt durch Verdünnung, insbesondere mit Wasser, auf die Messkonzentration in der Alkalität so stark abschwächt, daß durch Zugabe von Sodalösung, zweckmässig im Überschuß, die Bedingungen für die Bildung des Azofarbstoffes optimiert werden.

6. Verfahren nach Anspruch 1 bis 5, dadurch gekennzeichnet, daß man die Meßergebnisse mit Hilfe einer Eichkurve auswertet, die durch Lösen definierter Mengen von Guanin in der Weise erhalten wurde, wie sie erfindungsgemäß für die Behandlung der Staubprobe durchgeführt wird sowie Durchführung der Farbreaktion bei diesen Eichlösungen ebenfalls in der Weise, wie sie erfindungsgemäß für den aus den Staubproben erhaltenen Extrakt beschrieben wird.

**Claims**

1. Process for detecting the presence of allergen-containing house dust such as is produced during or after the appearence of house dust mites, in which samples of dust appearing in buildings are thoroughly suspended in aqueous-alcoholic alkali metal hydroxide solution, and a colour reaction is carried out with the resulting extract using an aromatic diazo compound, characterised in that in order to evaluate the allergological activity of the dust sample, quantitative measurement of the colour intensity of the colour reaction is carried out.

2. Process as claimed in claim 1, characterised in that the colour measurement is performed using a spectrophotometer.

3. Process as claimed in claim 1 and 2, characterised in that the dust sample used has previously been pre-treated with an aqueous-alcoholic mixture in order to purify it.

4. Process as claimed in claims 1 to 3, characterised in that the quantity of strongly alkaline solution used to extract the ingredients relevant to the detection is kept to a minimum in the interests of the subsequent development of colour.

5. Process as claimed in claim 1 to 4, characterised in that the strongly alkaline extract obtained according to claim 4 is so weakened in its alkalinity by dilution, particularly with water, to the concentration for measurement that the conditions for the formation of the azo dye are optimised by the addition of soda solution, expediently in excess.

6. Process as claimed in claim 1 to 5, characterised in that the results of the measurement are evaluated by means of a calibration curve obtained by dissolving defined amounts of guanine in the manner used according to the invention for treating the dust sample and carrying out the colour reaction with these calibrating solutions again in the manner described according to the invention for the extract obtained from the dust sample.

**Revendications**

1. Procédé pour la reconnaissance de la présence de poussière de maison porteuse d'allergènes telle qu'elle se manifeste au moment ou après l'apparition d'acariens de poussière de maison, dans lequel on met soigneusement en suspension des échantillons de poussière survenant dans les bâtiments dans une solution d'hydroxyde de métal alcalin hydro-alcoolique, effectue avec l'extrait obtenu une réaction colorée à l'aide d'un composé diazoïque aromatique, caractérisé en ce que, pour l'évaluation de l'activité allergologique de l'échantillon de poussière, on procède à une mesure quantitative de l'intensité de la teinte de la réaction colorée.

2. Procédé selon la revendication 1, caractérisé en ce que la mesure de la teinte s'effectue à l'aide d'un spectrophotomètre.

3. Procédé selon les revendications 1 et 2, caractérisé en ce que, en vue de la purification, l'échantillon de poussière mis en œuvre a été préalablement prétraité avec un mélange hydro-alcoolique.

4. Procédé selon les revendications 1 à 3, caractérisé en ce que, dans l'intérêt du développement de la teinte subséquente, on limite à la quantité minimale la solution fortement alcaline pour l'extraction des constituants intervenant dans la détection.

5. Procédé selon les revendications 1 à 4, caractérisé en ce qu'on atténue l'alcalinité de l'extrait fortement alcalin obtenu selon la revendication 4 par dilution, notamment avec de l'eau, jusqu'à la concentration de mesure si fortement que, par addition d'une solution de carbonate de soude, de préférence en excès, les conditions pour la formation du colorant azoïque sont optimisées.

6. Procédé selon les revendications 1 à 5, caractérisé en ce qu'on dépouille les résultats de mesure à l'aide d'une courbe d'étalonnage qui a été obtenue par dissolution de quantités définies de guanine telle qu'elle est réalisée, selon l'invention, pour le traitement de l'échantillon de poussière ainsi que la mise en œuvre de la réaction colorée avec ces solutions d'étalonnage, également selon la manière décrite conformément à l'invention à propos de l'extrait obtenu à partir des échantillons de poussière.